# EUROPEAN PATENT SPECIFICATION

(11) **EP 1 726 956 B1**
(45) Date of publication and mention of the grant of the patent: **28.07.2010**
(21) Application number: 04724410.8
(22) Date of filing: 30.03.2004
(51) Int. Cl.: G01N 33/543, G01N 27/327

(54) **BRASSIERE COMPRISING A DIAGNOSTIC SENSOR.**
BÜSTENHALTER MIT EINEM DIAGNOSTISCHEN SENSOR
SOUTIEN-GORGE AVEC UN CAPTEUR DE DIAGNOSTIC

(30) Priority: 26.02.2004 JP 2004050855
(43) Date of publication of application: 29.11.2006
(73) Proprietor: Seems Inc., Chiyoda-ku Tokyo 102-0093 (JP)
(72) Inventor: URUSHIHATA, Naoki, PIXEN, Inc., Tokyo 102-0093 (JP); YANAGAWA, Hideki, PIXEN, Inc., Tokyo 102-0093 (JP); TAJIMA, Yukinobu, PIXEN, Inc., Tokyo 102-0093 (JP)
(74) Representative: HOFFMANN EITLE
(86) International application number: PCT/JP2004/004524
(87) International publication number: WO 2005/083428

(56) References cited:
- WO-A-00/00233
- WO-A-00/32091
- WO-A-00/75792
- WO-A-98/27417
- WO-A-02/086149
- WO-A-03/102546
- WO-A1-94/13209
- WO-A1-99/67628
- GB-A- 2 203 250
- JP-A- 2002 311 025
- JP-A- 2002 350 317
- US-A1- 2002 094 531
- GARDNER J. & BARTLETT P.: "A brief history of electronic noses" SENSORS AND ACTUATORS B, vol. 18-19, 1994, pages 211-220, XP002451152

## Description

### Technical Field

The present invention relates to a brassiere comprising a sensor for detecting and diagnosing occurrence of abnormality in a living body.

### Background Art

Various techniques for detecting various diseases, illness, and other abnormalities of a body have been conventionally proposed. Such detecting techniques are roughly divided into techniques for removing tissues from a body by incision and techniques for detecting abnormality without incising the body.

When a physical burden on a subject as an object to be subjected to detection of the presence/absence of abnormality is considered, the abnormality detection without incising the body is preferable.

It is well known as an empirical rule that when something abnormal occurs in the body, odor which does not develop when a person is healthy develops.

However, the sense of smell is a particularly emotional or affective sense among the five senses of a human. A person may feel the same odor as comfortable "aroma" or "bad odor" depending on a slight difference in the atmosphere in which the odor exists. The sense of small largely varies among persons.

Consequently, techniques for detecting abnormality in a body using the odor as a reference have been hardly practically used.

A sensor capable of measuring an enzyme or protein specific to a disease by using an antibody-enzyme system has been also proposed (refer to, for example, Japanese Published Unexamined Patent Application No. H10-267888).

WO00/00233 discloses a disposable article comprising a sensor for detecting a marker in the wearer's faeces.

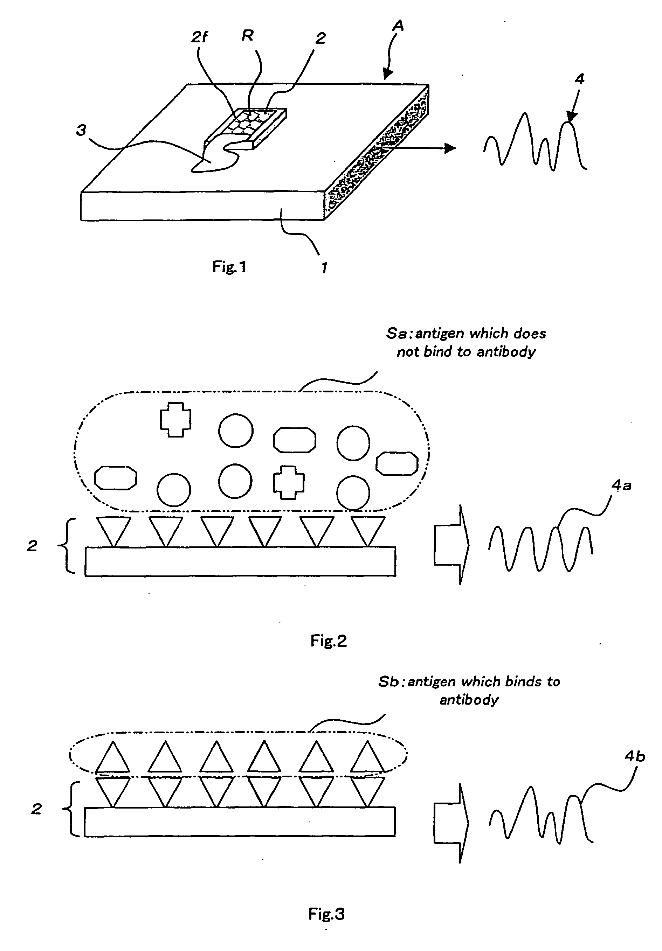

GB2203250 discloses a brassiere comprising a diagnostic sensor and a housing part (see figure 1 with sensors (2)). The diagnostic sensors are temperature sensors measuring the temperature of the breast. Output signals from the sensors (2) are carried by wires (6) to a microprocessor (5) (bridging sentence pages 5-6). Thus, the temperature sensor is composed of a detecting means and signal generating means. Said temperature sensor is regarded as a diagnostic sensor because in case of breast inflammation (mastitis) the temperature of the breast will rise and thereby detecting a pathological condition.

WO02/086149 discloses surface acoustic wave devices which have been used to detect volatiles in samples associated with a particular disease state.

WO9827417 describes biosensors which have attached antibodies on semiconductive substrate suitable for the quantification of analytes in medium.

It is, however, difficult to apply such a sensor to a method of detecting abnormality in a body using the odor as a reference.

### Disclosure of the Invention

An object of the present invention is to provide a brassiere comprising a diagnostic sensor capable of easily detecting abnormality in the wearer's body using the odor as a reference by a simple apparatus without removing a part of tissues and/or taking blood, which gives pain to the patient or without requiring a conventional inspection method of examining a part of removed tissues and/or blood under a microscope for long time.

The inventors have been paying attention to the phenomenon that when a living body has something abnormal or a disease, odor which does not develop when a person is healthy develops from the living body itself, body fluid such as blood, sweat, or the like of the living body, and gases emitted from the living body such as expired gas, inspired gas, water vapor from the skin, and the like.

This time, the inventors paid attention to the phenomenon that the odor is caused by molecules or particles of a pathogen or an antigen or ligand corresponding to abnormality, or disease, which are included in a part of the living body having abnormality or disease, a body fluid, and a gas from the living body. By detecting the pathogen, antibody, or ligand from the part, body fluid, or gas, the state of the living body can be diagnosed with high precision.

A brassiere of the present invention based on the finding includes the features of claim 1.

The pathogen, antigen, or ligand is a cause of a disease and, preferably, the detecting means does not detect all of pathogens or antigens but detects a specific pathogen, antigen, or ligand.

Preferably, the detecting means detects, for example, only one kind of pathogen, antigen, or ligand or has either an antibody which binds to the pathogen or antigen to be detected or a protein (ligand receptor) which binds to the ligand, and has, as a sensing part, a part having the antibody or protein. More preferably, a monoclonal antibody is used as the antibody.

In addition, the detecting means can be also constructed so as to detect body odor of each person corresponding to a gene cluster of a major histocompatibility complex (MHC) used when an immunocyte distinguishes between itself and a foreign matter.

In the brassiere of the present invention, the detecting means is formed by applying or attaching an antibody or ligand on or near an integrated circuit formed on a semiconductor substrate, a part to which the antibody is applied or attached and the integrated circuit are electrically connected to each other via a conductor, the signal generating means is formed in the integrated circuit, and when a surface acoustic wave current which is generated when the antibody and the antigen bind to each other or the protein and the ligand bind to each other is transmitted via the conductor to the integrated circuit, an electric signal corresponding to the surface acoustic wave current is transmitted to the outside of the integrated circuit.

The diagnostic system of the present invention comprising brassiere having the diagnostic sensor includes: signal amplifying means for amplifying a signal generated by the diagnostic sensor; storing means (database) for storing, as a base signal, a signal generated when the diagnostic sensor does not detect a pathogen, antigen, or ligand; control means

(control unit); and display means (monitor), wherein the control means compares a signal generated by the diagnostic sensor with the base signal stored in the storing means, thereby determining whether or not the signal generated by the diagnostic sensor is a signal generated in the case where a pathogen, antigen, or ligand is detected (claim 2).

The diagnostic system comprising the diagnostic sensor (sensor chip) and a computer network of the invention includes: reading means (reader) provided on the patient side, for reading a signal generated by the diagnostic sensor and generating an electric signal; a patient-side computer for sending the electric signal from the reading means to the network; and a diagnosing-side computer for receiving the signal from the reading means sent via the network. The diagnosing-side computer includes: storing means for pre-storing, as a base signal, a signal generated by the reading means in the case where the diagnostic sensor does not detect a pathogen, antigen, or ligand; comparing means for comparing the signal sent via the network with the base signal; and determining means for determining the state of the patient side corresponding to the signal sent via the network on the basis of an output signal of the comparing means (claim 3).

The brassiere of the present invention is formed in such a manner that it comprises a housing part for housing any one of the above-described diagnostic sensors, and in the case where abnormality occurs in a mamma, an antigen or ligand as a cause of the abnormality is detected by the diagnostic sensor in the housing part, and the abnormality is notified.

The antigen is, for example, an antigen specific to breast cancer.

Preferably, the housing part is provided in a position near a nipple.

The diagnostic sensor having a (specific part) detector (specific part) for detecting binding between an antibody and an antigen or the sensor for detecting binding between a ligand and a protein (ligand receptor) can be replaced with another sensor.

For example, the detection can be also performed by an odor sensor utilizing an odor sensing function, a sensor utilizing an organic dye film of petain dye, merocyanine dye, or the like, a sensor using a chemical material such as beta carotene, or a metal oxide semiconductor sensor made of tin oxide (SnO₂), zinc oxide (ZnO), iron oxide (Fe₂O₃), titanium oxide (TiO₃), or the like.

It is also possible to prepare a plurality of sensors and collate the correlation of the sensors with a prepared response pattern to specify a component related to any of various diseases from an approximated value.

### Brief Description of the Drawings

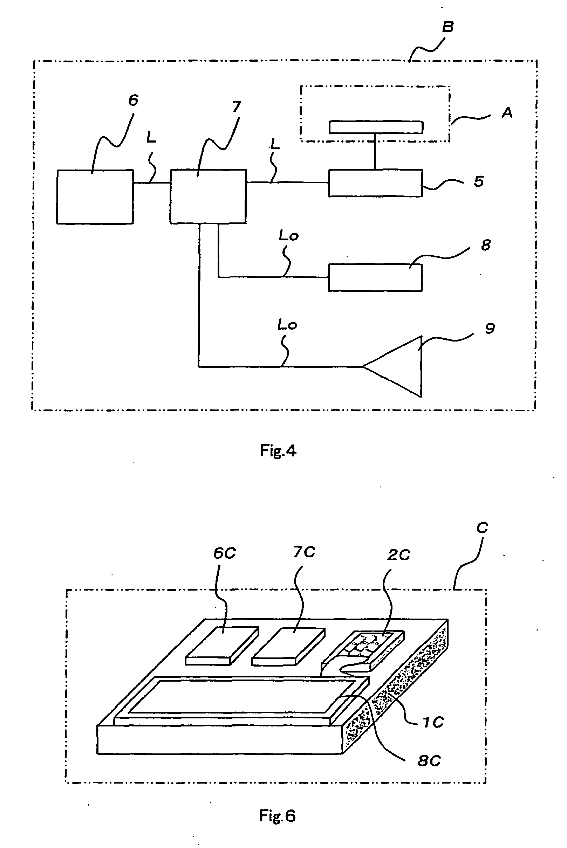

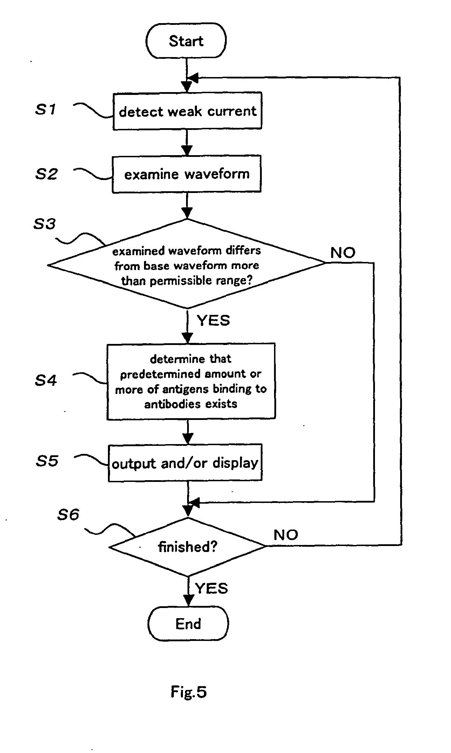

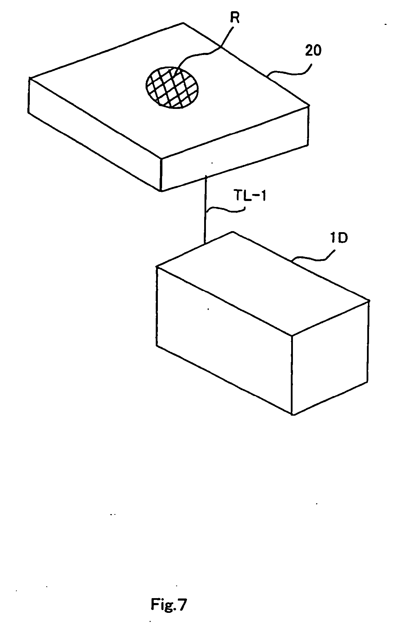

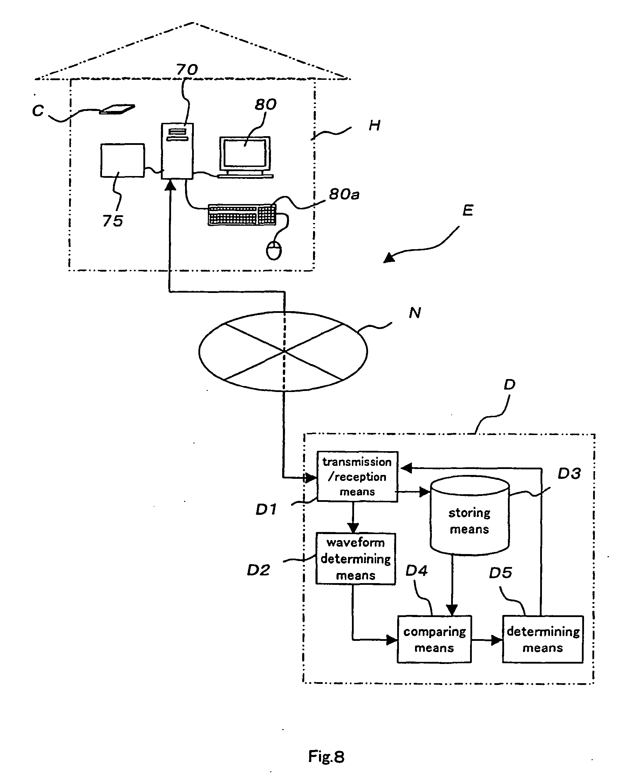

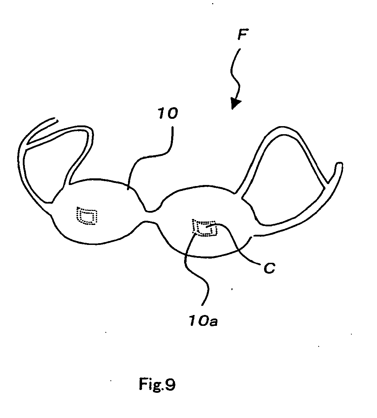

FIG. 1 is a perspective view showing a general configuration of a diagnostic sensor, FIG. 2 is a schematic diagram showing an image of a binding state between a monoclonal antibody and an antigen in the embodiment of Fig.1, in which the antigen does not bind to the antibody, FIG. 3 is a schematic diagram showing an image of a binding state between the monoclonal antibody and the antigen in the embodiment of Fig.1, in which the antigen binds to the antibody, FIG. 4 is a block diagram showing a general configuration of a diagnostic system , FIG. 5 is a flowchart showing a diagnosing method of the embodiment of Fig. 4, FIG. 6 is a perspective view showing the configuration of a diagnostic chip, FIG. 7 is a block diagram showing the configuration of a diagnostic chip , FIG. 8 is a block diagram showing the configuration of a diagnostic system , and FIG. 9 is a perspective view of a brassiere for diagnosing breast cancer of the invention.

### Best Mode for Carrying out the Invention

First, a first embodiment will be described with reference to FIGS. 1 to 3. FIGS. 1 to 3 are diagrams stereoscopically showing an example of a diagnostic sensor of the first embodiment.

In FIG. 1, a sensor indicated by reference character A is constructed by a monoclonal antibody R mounted on an integrated circuit 1 formed on a semiconductor substrate and an electric conductor 3 connecting the monoclonal antibody R and the semiconductor integrated circuit 1.

The sensor A has the antibody R (monoclonal antibody) as shown in FIG. 2. In the case where the antibody S in a detection part 2 as detecting means and an antigen S mounted on the top face of the detecting part 2 do not bind to each other (in the case where the antigen is Sa), a weak waveform (for example, sine wave) 4a as an electric signal detected does not change. However, as shown in FIG. 3, when the antibody R of the detecting part 2 and the antigen S bind to each other (in the case where the antigen is Sb), a current 4b called a surface acoustic wave current or an interface weak current is generated and the weak waveform 4a changes.

By comparing the changed weak waveform 4b with the weak waveform 4a generated in the case where the antibody R and the antigen S do not bind to each other (the antigen is Sa), whether the antibody R and the antigen S bind to each other or not is determined.

To assure stability of the antibody R, the detecting part 2 having the antibody R is covered with a film 2f. The film 2f is made of a material which transmits an antigen molecule or antigen particle but does not transmit the antibody.

For the detecting part 2 of the sensor chip, in place of the antibody R, a substance which reacts with a specific antigen S may be used.

Examples of the material which reacts with the specific antigen S are an odor sensor utilizing an odor sensing function, a sensor utilizing an organic dye film of petain dye, merocyanine dye, or the like, a sensor using a chemical material such as beta carotene, and a metal oxide semiconductor sensor of tin oxide (SnO₂), zinc oxide (ZnO), iron oxide (Fe₂O₃), titanium oxide (TiO₃), or the like. By any of the sensors, detection can be performed.

It is also possible to prepare a plurality of sensors and collate the correlation of the sensors with a prepared response pattern to specify a component related to any of various diseases from an approximated value.

Next, a second embodiment will be described with reference to FIGS. 4 and 5. The second embodiment of FIGS. 4 and 5 relates to a diagnostic system including the diagnostic sensor of the first embodiment.

First, referring to FIG. 4, the configuration of the diagnostic system will be described.

The diagnostic system indicated by reference character B has: the diagnostic sensor A constructed by the detecting part 2 having the antibody R (monoclonal antibody), the semiconductor integrated circuit 1, and the electric conductor 3 connecting the detecting part 2 and the semiconductor integrated circuit 1; a semiconductor amplifier (signal amplifier) 5 connected to the diagnostic sensor A and amplifying a signal generated from the diagnostic sensor A; and a control unit 7 connected to the signal amplifier 5 and a database 6 via a signal line L.

Further, the diagnostic system B includes an image display device (monitor) 8 and an audio output device 9. The image display device (monitor) 8 and the audio output device 9 are connected to the control unit 7 via a control signal line Lo.

Next, a diagnosing method carried out by the diagnostic system B will be described with reference to FIG. 5.

First, the control unit 7 detects a weak current output from the semiconductor amplifier 5 (step S1).

After that, the control unit 7 examines the waveform of the weak current (step S2) and determines whether the detected waveform is different from a base waveform more than a permissible range or not (step S3). The "base waveform" denotes here a waveform of a weak current generated in the case where the antibody R and the antigen S do not bind to each other.

If the waveform of the weak current is different from the base waveform more than the permissible range (YES in step S3), the control unit 7 moves to step S4. If the waveform of the weak current is not different from the base waveform more than the permissible range (NO in step S3), and the control unit 7 moves to step S6.

In step S4, the control unit 7 determines that "a predetermined amount or more of the antigens S binding to the antibody R exists", and notifies, for example, a doctor, a subject (patient), or the like who operates the diagnostic system B of the fact "a predetermined amount or more of the antigens binding to the antibody exists" by using the image display device 8 or the audio output means 9 (step S5).

In the following step S6, the control unit 5 determines whether the diagnosis (control) is finished or not. If the diagnosis is finished (YES in step S6), the control unit 7 finishes the control. On the other hand, when the control unit 7 determines that the test has not been finished (NO in step S6), the control unit 7 returns to step S1 and repeats step S1 and the subsequent steps.

With the diagnostic sensor A of the first embodiment and the diagnostic system B using the sensor A, by comparing a signal generated by the diagnostic sensor A with a base signal stored in the database 4, whether the signal generated by the diagnostic sensor A is a signal generated in the case where a pathogen or antigen is detected or not can be easily determined. Therefore, without removing a part of tissues and/or taking blood that gives pain to the patient, abnormality in the human body can be detected.

Referring now to FIG. 6, a third embodiment will be described. The third embodiment of FIG. 6 is obtained by forming the diagnostic system of the second embodiment of FIGS. 4 and 5 in a single chip.

Specifically, in the third embodiment, a single chip (diagnostic chip) C has thereon a semiconductor integrated circuit 1C, a detecting part 2C having a monoclonal antibody, a storage 6C, a control unit 7C, and a liquid crystal display 8C as display means which are formed on the semiconductor integrated circuit 1C. With the single chip C, diagnosis can be conducted.

FIG. 7 shows a fourth embodiment of the present invention.

In the first embodiment shown in FIGS. 1 to 3, the monoclonal antibody R is directly mounted on the integrated circuit 1. In the third embodiment of FIG. 6, the detecting part 2C having the antibody R is directly mounted on the integrated circuit 1C.

In contrast, in the fourth embodiment of FIG. 7, the monoclonal antibody R is mounted on a quartz resonator 20, and the quartz resonator 20 is electrically connected to an integrated circuit 1D via a signal transmission line TL-1. In other words, an electric signal generated by the quartz resonator 20 is transmitted to the integrated circuit 1D via the signal transmission line TL-1.

An electric change (fluctuations in the weak electric signal) which occurs when the antibody R (monoclonal antibody) and the antigen S bind to each other is amplified by the quartz resonator 20. The amplified signal is transmitted to the integrated circuit 1D via the signal transmission line TL-1.

In the integrated circuit 1D, for example, a signal process or information process is performed in a manner similar to the mode described above with reference to FIG. 5.

The other configuration and effects are similar to those of the first to third embodiments.

A fifth embodiment will be described with reference to FIG. 8.

In the third embodiment of FIG. 6, a diagnosis result (result of only "whether the antibody and the antigen bind to each other or not") is displayed on the chip. The details are unclear and it is difficult to carry out further diagnosis.

In contrast, the fifth embodiment of FIG. 8 relates to a diagnostic system (indicated by reference character E) in which a detection signal from the chip is received by signal reading means in a state where a predetermined part is detected by the chip. After the signal from the chip is received by the signal reading means, the signal is transmitted to, for example, diagnosis means in a medical facility via a network. By the diagnosis means, diagnosis is conducted on the basis of the signal from the chip.

In FIG. 8, a house H is provided with the diagnostic chip C similar to that in the third embodiment of FIG. 6 and a reader 75 for reading a diagnosis result of the chip C. The reader 75 is connected to a household personal computer 70. The personal computer 70 has a monitor 80 and input means 80a.

On the other hand, a computer D on the diagnosis side (medical institution) has transmission/reception means D1, waveform determining means D2, storing means D3, comparing means D4, and determining means D5.

The household personal computer 70 is connected to the transmission/reception means D1 of the diagnosis-side computer D via a network N, and sends a result (data of a weak waveform in which whether the antibody and the antigen bind to each other is reflected) obtained by the diagnosis chip C to the comparing means D4 via the transmission/reception means D1 and the waveform determining means D4.

The comparing means D4 compares data of various antibodies R stored in the storing means D3 with the waveforms obtained by the household chip C to determine if the subject providing data has a disease, illness, or problem on the basis of the obtained antibody data.

That is, the diagnosis itself is conducted in a manner similar to that of the second embodiment of FIGS. 4 and 5.

In the diagnostic system E having the diagnostic sensor (chip C) and the computer network N, by installing the diagnostic sensor (chip C) in the house H, information is automatically sent from a computer 50 in the house to the computer D on the diagnosis side via the network N. By collating and comparing the information with data prepared in the storing means D3 of the computer D on the diagnosis side, abnormality or disease can be determined in short time. Thus, the diagnostic system E contributes to early treatment.

Next, a sixth embodiment will be described with reference to FIG. 9.

The sixth embodiment of FIG. 9 is an embodiment (diagnostic apparatus F) in which the first embodiment of FIGS. 1 to 3, the second embodiment of FIGS. 4 and 5, the chip of the third embodiment of FIG. 6, and the fifth embodiment of FIG. 8 are used for particularly detecting breast cancer.

As shown in FIG. 9, the diagnostic apparatus F is obtained by forming a pocket part (housing part) 10a in a female brassiere 10 and housing the chip C in the pocket part 10a.

Since 90% or higher of breast cancer is "ductal carcinoma" starting from glandular epithelia of the ducts, particularly, to detect breast cancer, it is preferable to provide the pocket in a part where a nipple is positioned.

By disposing the chip C near a nipple, abnormality in the ducts can be detected early. Therefore, early detection of breast cancer can be performed.

It is also possible to provide the pocket 10a in another part of the brassiere 10 and house the chip C in the pocket 10a.

Although the sixth embodiment is directed to the female brassiere 10, it can be also applied to female panties (not in accordance with the invention). In this case, early detection of particularly, cancers in urinary organs, uterine cancer, and the like can be realized.

It is also possible to detect body odor, secreted material from the skin or its gas, or bad breath near cancer tissues of gastric cancer, liver cancer, or the like, thereby predicting occurrence of cancer.

In the first to sixth embodiments, it is also possible to provide protein (ligand receptor) in place of the antibody R (monoclonal antibody) and detect that a specific ligand selectively binds to the protein. In other words, in the embodiments, the antibody R can be replaced with protein as a ligand receptor, and the antigen S can be replaced with a ligand.

When the protein binds to the ligand, strong selectivity equivalent to binding between the antibody R and the antigen S is displayed. It can be regarded that the behavior in the case where the protein binds to a ligand is similar to that in the case where the antibody R binds the antigen S.

The embodiments shown in the diagrams are only illustrative but do not limit the technical range of the present invention.

For example, in the illustrated embodiments, each of the sensor chips A and C is constructed by combining the semiconductor 1 and the antibody R. A potential change may be detected and amplified by a quartz resonator sensor.

The diagnostic sensor having a (specific part) detector (specific part) for detecting binding between an antibody and an antigen can be replaced with another sensor.

For example, the detection can be performed by an odor sensor utilizing an odor sensing function, a sensor utilizing an organic dye film of petain dye, merocyanine dye, or the like, a sensor using a chemical material such as beta carotene, or a metal oxide semiconductor sensor of tin oxide (SnO₂), zinc oxide (ZnO), iron oxide (Fe₂O₃), titanium oxide (TiO₃).

Although a single kind of an antibody is provided for a sensor chip in the illustrated embodiments, a plurality of antibodies can be provided. Alternately, data for diagnosis can be detected with a plurality of kinds of sensors having different antibodies.

In the case of using a plurality of kinds of sensors, determination closer to the sense of humans can be made using a pattern of a figure obtained by totaling (or multiplexing) sensor outputs.

A diagnosing method using the diagnostic sensor of the present invention includes: a step (step S1 in FIG. 5) of detecting the weak current 4 generated from the semiconductor integrated circuit 1; a step (steps S2 and S3 in FIG. 5) of examining the waveform of the weak current and determining whether the detected waveform is different from a base waveform (a waveform of a weak current generated in the case where the antibody and the antigen do not bind to each other) more than a permissible range or not; and a step (steps S4 and S5 in FIG. 5) of displaying the result when a predetermined amount or more of antigens binding to the antibodies exists.

### Effects of the Invention

In the diagnostic sensor (A: claims 1 and 2) of the present invention or the diagnostic system (B: claim 3) using the diagnostic sensor, an electric change is generated on detection of a pathogen, antigen (S), or ligand as a cause of odor which develops when abnormality occurs. Consequently, by examining a signal from the sensor, the presence or absence of abnormality can be easily determined. That is, abnormality in a human body can be detected without removing a part of tissues and/or taking blood which gives pain to the patient.

In the diagnostic system (E: claim 4) of the present invention using the diagnostic sensor (C) ad the computer network (N), by installing the diagnostic sensor (C) in a house (H), information is automatically sent to the diagnosis-side computer (D) and is compared with data (D3) prepared in the diagnosis-side computer (D), thereby enabling abnormality or disease to be determined in short time. Thus, the system contributes to early treatment.

Further, when the user wears the brassiere (10: claim 1) for detecting breast cancer of the present invention, early finding of breast cancer can be realized naturally and easily without aware of a physical burden of a test.

## Claims

1. A brassiere comprising a diagnostic sensor and a housing part accommodating the diagnostic sensor , the diagnostic sensor including:
detecting means for detecting any of various pathogens existing in a part of the wearer's body and/or in a body fluid of the wearer's body or in a gas emitted from the wearer's body, or an antigen or ligand corresponding to an abnormality or disease, and
signal generating means for generating a signal when the detecting means detects any of the pathogens, antigen, or ligand, wherein the detecting means is formed by applying or attaching an antibody or protein on or near an integrated circuit formed on a semiconductor substrate,
a part to which the antibody or protein is applied or attached and the integrated circuit are electrically connected to each other via a conductor,
the signal generating means is formed in the integrated circuit, and
when a surface acoustic wave current, which is generated when the antibody and the antigen bind to each other or the protein and the ligand bind to each other, is transmitted via the conductor to the integrated circuit, a signal corresponding to the surface acoustic wave current is transmitted to the outside of the integrated circuit.

2. A diagnostic system comprising the brassiere according to claim 1, the system further comprising:
signal amplifying means for amplifying a signal generated by the diagnostic sensor;
storing means for storing, as a base signal, a signal generated when the diagnostic sensor does not detect a pathogen, antigen, or ligand;
control means; and
display means,
wherein the control means compares a signal generated by the diagnostic sensor with the base signal stored in the storing means, thereby detecting whether or not the signal generated by the diagnostic sensor is a signal generated in the case where a pathogen, antigen, or ligand is detected.

3. A diagnostic system comprising the brassiere according to claim 1 and a computer network , the system further comprising:
reading means provided on a patient side, for reading a signal generated by the diagnostic sensor and generating an electric signal;
a patient-side computer for sending the electric signal from the reading means to the network; and
a diagnosing-side computer for receiving the signal from the reading means sent via the network,
wherein the diagnosing-side computer comprises:
storing means for pre-storing, as a base signal, a signal generated by the reading means in the case where the diagnostic sensor does not detect a pathogen, antigen, or ligand;
comparing means for comparing the signal sent via the network with the base signal; and
determining means for determining the state of the patient side corresponding to the signal sent via the network on the basis of an output signal of the comparing means.

## Patentansprüche

1. Büstenhalter, umfassend einen Diagnosesensor und einen Gehäuseteil, der den Diagnosesensor aufnimmt, wobei der Diagnosesensor enthält:
ein Detektionsmittel zum Detektieren eines von verschiedenen Pathogenen, die in einem Teil des Körpers des Benutzers und/oder in einer Körperflüssigkeit des Körpers des Benutzers oder in einem Gas, das aus dem Körper des Benutzers emittiert wird, existieren, oder eines Antigens oder Liganden entsprechend einer Abnormalität oder Krankheit, und
ein Signalerzeugungsmittel zum Erzeugen eines Signals, wenn das Detektionsmittel eins der Pathogene, des Antigens oder Liganden detektiert, wobei das Detektionsmittel durch Anlegen der Anbringen eines Antikörpers oder Proteins an oder in der Nähe eines integrierten Schaltkreises gebildet ist, der auf einem Halbleitersubstrat ausgebildet ist,
wobei ein Teil, auf dem der Antikörper oder das Protein aufgebracht oder daran befestigt ist, und der integrierte Schaltkreis über einen Leiter miteinander elektrisch verbunden sind,
wobei das Signalerzeugungsmittel in dem integrierten Schaltkreis ausgebildet ist, und
wenn ein akustischer Oberflächenwellenstrom, der erzeugt wird, wenn der Antikörper und das Antigen aneinander binden oder das Protein und der Ligand aneinander binden, über den Leiter zu dem integrierten Schaltkreis übertragen wird, wobei ein Signal, das dem akustischen Oberflächenwellenstrom entspricht, zu der Außenseite des integrierten Schaltkreises übertragen wird.

2. Diagnosesystem, umfassend den Büstenhalter nach Anspruch 1, wobei das System ferner umfasst:
ein Signalverstärkungsmittel zum Verstärken eines Signals, das durch den Diagnosesensor erzeugt wird;
ein Speichermittel zum Speichern eines Signals, das erzeugt wird, wenn der Diagnosesensor kein Pathogen, Antigen oder Ligand detektiert, als ein Basissignal;
ein Steuerungsmittel; und
ein Anzeigemittel,
wobei das Steuerungsmittel ein Signal, das durch den Diagnosesensor erzeugt wurde, mit dem Basissignal vergleicht, das in dem Speichermittel gespeichert ist, wodurch detektiert wird, ob das durch den Diagnosesensor erzeugte Signal ein Signal ist, das in dem Fall erzeugt wird, wenn ein Pathogen, Antigen oder Ligand detektiert wird, oder nicht.

3. Diagnosesystem, umfassend den Büstenhalter nach Anspruch 1 und ein Computernetzwerk, wobei das System ferner umfasst:
ein Lesemittel, das auf einer Patientenseite vorgesehen ist, um ein Signal zu lesen, das durch den Diagnosesensor erzeugt wurde, und ein elektrisches Signal zu erzeugen;
einen patientenseitigen Computer zum Senden des elektrischen Signals von dem Lesemittel zu dem Netzwerk; und
einen diagnoseseitigen Computer zum Empfangen des Signals von dem Lesemittel, das über das Netzwerk gesendet wurde,
wobei der diagnoseseitige Computer umfasst:
ein Speichermittel zum vorherigen Speichern eines Signals, das durch das Lesemittel in dem Fall erzeugt wurde, wo der Diagnosesensor kein Pathogen, Antigen oder Ligand detektiert, als ein Basissignal;
ein Vergleichsmittel zum Vergleichen des Signals, das über das Netzwerk gesendet wurde, mit dem Basissignal; und
ein Bestimmungsmittel zum Bestimmen des Zustands der Patentenseite entsprechend dem Signal, das über das Netzwerk gesendet wurde, auf der Basis eines Eingabesignals des Vergleichsmittels.

## Revendications

1. Soutien-gorge comprenant un capteur de diagnostic et une partie de boîtier logeant le capteur de diagnostic, le capteur de diagnostic comportant:
un moyen de détection pour détecter l'un quelconque parmi divers pathogènes qui se trouvent dans une partie d'un corps du porteur et/ou dans un fluide corporel du corps du porteur ou dans un gaz émis du corps du porteur, ou un antigène ou un ligand correspondant à une anomalie ou à une maladie, et
un moyen de génération de signal pour générer un signal lorsque le moyen de détection détecte l'un quelconque parmi les pathogènes, l'antigène, ou le ligand, où le moyen de détection est formé en appliquant ou en fixant un anticorps ou une protéine sur ou prés d'un circuit intégré formé sur un substrat semi-conducteur,
une partie à laquelle l'anticorps ou la protéine est appliqué(e) ou fixé(e) et le circuit intégré sont électriquement reliés les uns aux autres à travers un conducteur ,
le moyen de génération de signaux est formé dans le circuit intégré, et
lorsqu'un courant d'ondes acoustiques de surface, qui est généré lorsque l'anticorps et l'antigène liés l'un à l'autre ou la protéine et le ligand sont liés l'un à l'autre, est transmis à travers le conducteur au circuit intégré, un signal correspondant au courant d'ondes acoustiques de surface est transmis à la partie extérieure du circuit intégré.

2. Système de diagnostic comprenant le soutien-gorge selon la revendication 1, le système comprenant en outre:
un moyen d'amplification de signaux pour amplifier un signal généré par le capteur de diagnostic;
un moyen de stockage pour stocker, comme signal de base, un signal généré lorsque le capteur de diagnostic ne détecte pas de pathogène, d'antigène, ou de ligand;
un moyen de commande; et
un moyen d'affichage,
où le moyen de commande compare un signal généré par le capteur de diagnostic au signal de base stocké dans le moyen de stockage, détectant ainsi si le signal généré par le capteur de diagnostic est un signal généré dans le cas où un pathogène, un antigène, ou un ligand est détecté ou non.

3. Système de diagnostic comprenant le soutien-gorge selon la revendication 1 et un réseau informatique, le système comprenant en outre:
un moyen de lecture pourvu côté patient, pour lire un signal généré par le capteur de diagnostic et générer un signal électrique;
un ordinateur côté patient pour envoyer le signal électrique du moyen de lecture au réseau ; et
un ordinateur côté diagnostic pour recevoir le signal du moyen de lecture envoyé à travers le réseau,
où l'ordinateur côté diagnostic comprend:
un moyen de stockage pour pré-stocker, comme signal de base, un signal généré par le moyen de lecture dans le cas où le capteur de diagnostic ne détecte pas de pathogène, d'antigène, ou de ligand;
un moyen de comparaison pour comparer le signal envoyé à travers le réseau au signal de base; et
un moyen de détermination pour déterminer l'état côté patient correspondant au signal envoyé à travers le réseau sur la base d'un signal de sortie du moyen de comparaison.
